# FASCICULE DE BREVET EUROPEEN

(11) **EP 1 648 431 B1**
(45) Date de publication et mention de la délivrance du brevet: **03.06.2009**
(21) Numéro de dépôt: 04767761.2
(22) Date de dépôt: 22.07.2004
(51) Int. Cl.: A61K 31/132, A23L 1/305, A61P 25/00, A61P 31/12, A61P 9/10

(54) **UTILISATION D'UNE COMPOSITION PAUVRE EN POLYAMINES POUR LA REALISATION D'UN ALIMENT THERAPEUTIQUE HUMAIN**
NEUE VERWENDUNG EINER POLYAMIN-ARMEN ZUSAMMENSETZUNG ZUR HERSTELLUNG EINES MEDIZINISCHEN NAHRUNGSMITTELS FÜR DEN MENSCHLICHEN VERZEHR
NOVEL USE OF A POLYAMINE-POOR COMPOSITION FOR THE PRODUCTION OF A MEDICAL HUMAN FOOD

(30) Priorité: 31.07.2003 FR 0309480
(43) Date de publication de la demande: 26.04.2006
(73) Titulaire: Universite de Rennes 1, 35065 Rennes (FR); Universite victor Segalen Bordeaux II, 33076 Bordeaux Cedex (FR)
(72) Inventeur: MOULINOUX, Jacques-Philippe, F-35000 Rennes (FR); SIMONNET, Guy, F-33200 Bordeaux (FR)
(74) Mandataire: Larcher, Dominique
(86) Numéro de dépôt international: PCT/FR2004/001962
(87) Numéro de publication internationale: WO 2005/020974

(56) Documents cités:
- EP-A- 1 085 011
- WO-A-95/00041
- WO-A-95/00042
- WO-A-03/051348
- US-A- 6 114 392
- KERGOZIEN S ET AL: "Polyamine deprivation provokes an antalgic effect" LIFE SCIENCES, vol. 58, no. 24, 1996, pages 2209-2215, XP002309789 ISSN: 0024-3205
- GILAD G M ET AL: "EARLY POLYAMINE TREATMENT ENHANCES SURVIVAL OF SYMPATHETIC NEURONS AFTER POSTNATAL AXONAL INJURY OR IMMUNOSYMPATHECTOMY" DEVELOPMENTAL BRAIN RESEARCH, vol. 38, no. 2, 1988, pages 175-181, XP002271146 & ISSN: 0165-3806
- GILAD G M ET AL: "TREATMENT WITH POLYAMINES CAN PREVENT MONOSODIUM GLUTAMATE NEUROTOXICITY IN THE RAT RETINA" LIFE SCIENCES, vol. 44, no. 25, 1989, pages 1963-1969, XP002271147 & ISSN: 0024-3205
- GILAD G M ET AL: "Novel polyamine derivatives as neuroprotective agents." THE JOURNAL OF PHARMACOLOGY AND EXPERIMENTAL THERAPEUTICS. OCT 1999, vol. 291, no. 1, octobre 1999 (1999-10), pages 39-43, XP002271148 ISSN: 0022-3565

## Description

L'invention concerne le domaine pharmaceutique.

Plus précisément, l'invention concerne l'utilisation nouvelle de compositions alimentaire pauvres en polyamines pour la réalisation d'un aliment susceptible d'avoir des effets thérapeutiques.

Les récepteursN-méthyl-D-aspartate (NMDA) localisés au niveau des synapses des neurones sont composés de sept sous-unités connues, à savoir : NR1, NR2a, NR2b, NR2c, NR2d, NR3a, NR3b.

Il a été découvert que les récepteurs NMDA, et notamment la sous-unité NR2B, jouent un rôle critique dans de nombreux types de pathologies d'origine neuronale. La stimulation de ces récepteurs provoque en effet l'apoptose neuronale, et induit de ce fait des maladies neuro-dégénératives. Ces récepteurs sont aussi impliqués dans la perception de la douleur (voir : J.M Loftis, A. Janowsky / Phamacology & Therapeutics 97 (2003) 55-85).

L'utilisation clinique d'antagonistes des récepteurs NMDA est toutefois d'un intérêt très limitée du fait de l'induction d'effets secondaires psychotomimétiques (hallucinations, troubles mnésiques et d'apprentissage, troubles psychomoteurs....).

L'objectif de la présente invention est de proposer une alternative à l'utilisation de tels antagonistes, pour inhiber efficacement le fonctionnement de la sous-unité NR2B des récepteurs NMDA, c'est-à-dire sans induire d'effet indésirable majeur.

Un autre objectif de la présente invention est de proposer un agent thérapeutique permettant de lutter contre le développement d'une sensibilisation à la douleur, contre la mémorisation de la douleur et par voie de conséquence contre la chronicisation de la douleur.

Notamment un objectif de la présente invention est de proposer un agent thérapeutique permettant, le cas échéant, de restaurer les effets analgésiques des substances opïoides en s'opposant au processus de tolérance.

Les opioïdes, telle la morphine, sont des analgésiques puissants dont l'utilisation est largement répandue. Toutefois, ils provoquent aussi, de manière dose-dépendante, le développement d'une hypersensibilité à la douleur à long terme (effet dit pronociceptif) conduisant à des hyperalgésies (sensation douloureuse exagérée à un stimulus nociceptif) et des allodynies (sensation ressentie comme douloureuse à un stimulus non nociceptif) de longue durée. Cette augmentation de sensibilité à la douleur pourrait rendre compte de l'apparition d'une tolérance aux effets analgésiques (Simonnet et al., NeuroReport, 2003, 14, 1-7).

Ces objectifs sont atteints grâce à l'invention qui concerne l'utilisation d'une composition alimentaire à usage humain présentant moins de 1600 picomoles de polyamines pour la réalisation d'un aliment thérapeutique destiné à lutter contre un syndrome dans lequel la sous-unité NR2-B du récepteur N-méthyl-D-aspartate est impliquée et choisi dans le groupe constitué par :
- la sensibilisation, la mémorisation et par voie de conséquence la chronicisation de la douleur,
- la tolérance aux effets analgésiques des analgésiques opioïdes,
- la dépendance vis-à-vis de substances à potentialité toxicomanogène,
ladite composition contenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

On notera que de telles compositions alimentaires à usage humain sont connues en tant que telles et décrites dans WO-9500041 au nom de la Demanderesse.

Dans le cadre de WO-9500041, ces compositions sont utilisées comme agent anti-cancéreux (notamment pour le cancer de la prostate), pour stimuler le système immunitaire, pour stimuler l'activité des cellules NK, pour stimuler la production endogène d'interleukine-2, comme agent antalgique et comme agent visant à réduire l'appétit.

De telles compositions alimentaires pourront être administrées par voie entérale, c'est-à-dire par la bouche , mais aussi par voir parentérale par exemple à l'aide d'une sonde.

On rappelle que les polyamines et tout particulièrement la putrescine (I), la spermidine (II) et la spermine (III) sont présentes dans toutes les cellules.

⁺NH₃ - (CH₂)₄ - NH₃⁺ (I)

⁺NH₃ - (CH₂)₃ - NH - (CH₂)₄ - NH₃⁺ (II)

⁺NH₃ - (CH₂)₃ - NH - (CH₂)₄ - NH - (CH₂)₃ - NH₃⁺ (III)

Bien que ces molécules aient été longtemps considérées comme dénuées de tout rôle physiologique et ne représentant qu'une étape terminale du catabolisme tissulaire, de nombreux travaux scientifiques ont montré que les polyamines issues de la décarboxylation de l'ornithine étaient en fait des molécules biologiquement actives et capables d'intervenir à différents niveaux importants de la vie de la cellule.

Ces molécules que l'on trouve non seulement à l'intérieur même des cellules mais également à l'état circulant dans les liquides biologiques de l'organisme, tels que le sang sont issues de trois sources principales :
- la prolifération cellulaire physiologique (croissance et/ou renouvellement des cellules constitutives de l'organisme) et tumorale,
- l'alimentation,
- les bactéries intestinales.

Différents travaux ont par ailleurs mis en évidence que, chez l'animal, l'administration conjointe :
- d'une alimentation dépourvue de polyamines,
- d'α-DFMO,
- d'un inhibiteur de la polyamine-oxydase (PAO) supprimant la rétroconversion oxydative de la spermidine et de la spermine en putrescine, et
- de néomycine et de métronidazole,
entraîne une inhibition quasi-totale de la progression tumorale du carcinome pulmonaire de Lewis 3LL (Seiler N. et al, Cancer Research, 1990, n°50, pp. 5077-5083), du glioblastome humain U251 (Moulinoux J-Ph. et al, Anticancer Research, 1991, n°11, pp. 175-180), de l'adénocarcinome de la prostate Dunning MAT-LyLu (Moulinoux J-Ph. et al, Journal of Urology, 1991, n° 146, pp. 1408,1412) et du neuroblastome humain neuro 2a (Quemener et al, "Polyamines in the gastro-intestinal tract", Dowling R.H., Fölsch I.R. et Löser C Ed., Kluwer Academic Publishers Boston, 1992, pp. 375-385).

Il a par ailleurs été également démontré chez l'animal que la déplétion en polyamines pouvait considérablement potentialiser les effets antiprolifératifs des drogues antitumorales conventionnelles (méthotrexate, cyclophosphamide, vindesine) tout en allongeant le temps de survie des animaux et pouvait permettre de réduire les quantités de drogues administrées tout en conservant le même effet antitumoral (Quemener V. et al, "Polyamine deprivation enhances antitumoral efficacy of chemotherapy", Anticancer Research n°12, 1992, pp. 1447-1454).

La présente invention vise donc à couvrir une nouvelle utilisation de telles compositions alimentaires, utilisation non évidente au vu de l'art antérieur, à savoir la lutte contre les syndromes ou pathologies dans lesquels la sous-unité NR2-B du récepteur N-méthyl-D-aspartate est impliquée.

Au niveau du système nerveux, les polyamines sont capables d'agir (entre autres effets) sur un site membranaire localisé sur la sous-unité NR1 des récepteurs N-méthyl-D-aspartate (NMDA). En activant ce site récepteur (sites polyamines), les polyamines lèveraient l'inhibition exercée par un site sensible aux protons localisé également sur la sous-unité NR1 (proton sensor H⁺) lui-même inhibiteur du fonctionnement du récepteur-canal NMDA. Cette levée d'inhibition permettrait les interactions allostériques entre les sous-unités NR1 et NR2B permettant un bon fonctionnement des récepteurs NMDA (Traynelis et al., Science, 1995, 268, 873-876)

Parmi les syndromes et pathologies dans lesquels la sous-unité NR2-B du récepteur N-méthyl-D-aspartate est impliquée, on peut citer :
- la sensibilisation et la mémorisation de la douleur, et corollairement sa chronicisation ;
- la tolérance aux effets analgésiques des analgésiques opioïdes tels que la morphine et les morphinomimétiques ;
- la dépendance vis-à-vis de différentes substances à potentialité toxicomanogène (alcool, tabac, drogues...) et les comportements compulsifs en résultant ;

La présente invention est donc susceptible d'être mise en oeuvre pour traiter ces pathologies ou syndromes.

Préférentiellement, la composition utilisée selon la présente invention contient moins d'environ 400 picomoles/g de putrescine, moins d'environ 400 picomoles/g de spermidine, moins d'environ 400 picomoles/g de spermine et moins d'environ 400 picomoles/g de cadavérine.

Préférentiellement, la composition utilisée selon la présente invention contient moins d'environ 400, préférentiellement moins d'environ 200, picomoles/g de polyamines.

Avantageusement, la composition utilisée selon la présente invention contient moins d'environ 100, préférentiellement moins d'environ 50, picomoles/g de putrescine, moins d'environ 100, préférentiellement moins d'environ 50, picomoles/g de spermidine, moins d'environ 100, préférentiellement moins d'environ 50, picomoles/g de spermine et moins d'environ 100, préférentiellement moins d'environ 50, picomoles/g de cadavérine. Une telle composition apporte, journellement, au moins 17 fois moins de putrescine, 40 fois moins de cadavérine, 70 fois moins de spermidine et 220 fois de spermine que l'alimentation naturelle humaine la plus pauvre qui soit en teneur de polyamines, mais qui réponde néanmoins aux besoins nutritionnels humains.

Une telle composition pourra être présentée sous forme sèche à dissoudre extemporanément dans un véhicule neutre ou sous forme liquide prête à l'emploi. Dans tous les cas, la composition est présentée sous forme stérile.

Une telle composition est particulièrement bien adaptée à l'homme et constitue un substitutif alimentaire qui permet de carencer les patients de façon efficace en polyamines. Une telle composition permet en effet d'alimenter un patient de façon satisfaisante tout en induisant une carence en polyamines, d'une part en inhibant la synthèse intracellulaire de polyamines et d'autre part en diminuant l'apport de polyamines exogènes.

Une telle composition permet d'inhiber fortement la synthèse endogène des polyamines et diminue de façon très importante l'apport en ces composés puisque les différents ingrédients qui la constituent en sont quasiment dépourvus. Afin de diminuer également les apports en polyamines par les bactéries intestinales, cette composition pourra être administrée concomitamment à une décontamination du tractus gastro-intestinal au moyens d'antibiotique(s) et/ou d'antiparasitaire(s), tels que, par exemple, la néomycine et le métronidazole. On pourra d'ailleurs envisager d'inclure de tels antibiotique(s) et/ou antiparasitaire(s) directement dans ladite composition, sans sortir du cadre de l'invention.

Les nutriments utilisés dans la composition alimentaire selon l'invention possèdent une bonne valeur nutritionnelle même chez les sujets malades.

La quantité d'eau employée pour réaliser la composition utilisée selon la présente invention est déterminée pour que la composition soit plus ou moins liquide et puisse être facilement ingérée par le patient.

Le pourcentage pondéral du mélange constitué de vitamines, de minéraux et d'électrolytes est choisi de façon à répondre aux proportions, connues de l'homme du métier, devant se trouver dans une alimentation équilibrée.

Préférentiellement, la composition utilisée selon la présente invention contient moins de 100 picomoles/g de putrescine, moins de 100 picomoles/g de spermidine, moins de 100 picomoles/g de spermine, moins de 100 picomoles/g de cadavérine.

Une telle composition pourra être administrée conjointement à au moins un inhibiteur de la synthèse intracellulaire des polyamines.

Selon une variante intéressante de l'invention la composition utilisée selon la présente invention est enrichie avec au moins un inhibiteur de la synthèse intracellulaire des polyamines à raison d'au plus 15% en poids par rapport au poids sec total de la composition et, préférentiellement, à raison d'une quantité comprise entre 0,2% et 7% en poids.

Les inhibiteurs de l'ODC utilisables sont choisis en particulier parmi les composés suivants :
Antagonistes du phosphate de pyridoxal
   .L-canaline
   . N-(5'-phosphopyridoxyl)ornithine
Inhibiteurs compétitifs
   . alpha-hydrazino-ornithine
   . acide DL-alpha-hydrazino-delta-aminovalérique
   . alpha-méthylornithine
   . trans-3-déhydro-DL-ornithine
   .1,4-diamino-trans-2-butène
   . 1,4-diaminobutanone
   . rétinol, rétinoïdes, b-carotènes
   . polyphénols
   . géraniol
   . terpènes
   . flavonoides
   . procyanidines
   . resveratrol

Inhibiteurs diaminés
   - 1,3-diaminopropane
   - 1,3-diamino-2-propanol
   - bis(éthyl)spermine
   - guanidinobutylamine
Inhibiteurs suicides et irréversibles
   - 2-difluorométhylomithine (DFMO)
   - monofluorométhylornithine
   - 2-monofluorométhyldéhydro-ornithine
   - 2-monofluorométhyldéhydro-ornithine méthyl ester
   - 5-hexyne-1,4-diamine
   - trans-hex-2-èn-5-yne-1,4-diamine
   - monofluorométhylputrescine
   - difluorométhylputrescine
   - alpha-allénylputrescine
   - (2R,5R)-6-heptyne-2,5-diamine.

Parmi ces inhibiteurs, les inhibiteurs compétitifs sont particulièrement préférés et notamment l'alpha-méhtylornithine (alpha-MO).

L'alpha-méthylornithine montre de nombreux avantages dans le cadre de l'utilisation proposée ici. En effet, l'alpha-MO présente l'intérêt d'être un composé naturel facilement synthétisable et a une constante d'inhibition élevée.

L'alpha-MO présente l'avantage d'être un simple inhibiteur compétitif de l'ornithine décarboxylase et diminue fortement les risques d'accoutumance de l'organisme par mutation aboutissant à une résistance cellulaire accrue.

Selon une variante, l'utilisation de la composition selon l'invention est enrichie en vitamines, notamment celles apportées, chez l'être humain sain, par les bactéries intestinales. En effet, l'antibiothérapie qui peut accompagner l'administration de ladite composition peut aussi conduire à diminuer l'apport en certaines vitamines. Dans ce cas, il peut s'avérer nécessaire d'enrichir la composition utilisée en ces vitamines afin de ne pas provoquer de carence vitaminique à la suite de l'administration prolongée de ladite composition. Notamment, il pourra s'avérer utile d'enrichir la composition en vitamines ou en dérivés de vitamines. Certains dérivés de la vitamine A (acide rétinoïque) sont en effet des inhibiteurs de l'activité ODC.

Préférentiellement, les glucides de la composition utilisée appartiennent au groupe comprenant les polymères de glucose, les maltodextrines, le saccharose, les amidons modifiés, le glucose monohydraté, le sirop de glucose déshydraté, le monostéarate de glycérol et leurs mélanges. De tels glucides sont en effet digestibles même en cas de pathologie digestive.

Selon une variante de l'invention, les protéines utilisées appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélanges.

Préférentiellement, les lipides appartiennent au groupe comprenant l'huile de beurre, l'huile d'arachide, les triglycérides à chaîne moyenne, l'huile de pépins de raisin, l'huile de soja, l'huile d'onagre et leurs mélanges. Avantageusement, lesdits lipides sont constitués par un mélange d'au moins une huile d'origine animale, d'au moins une huile d'origine végétale et de stéarate de glycérol.

Selon une variante de l'invention la composition utilisée selon la présente invention constitue une ration journalière alimentaire d'un être humain et comprend :
- éventuellement ledit inhibiteur de la synthèse intracellulaire des polyamines à raison de moins de 50g et préférentiellement à raison de 1 à 10 g,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

Les quantités de vitamines, de minéraux et d'électrolytes utilisés sont connues de l'homme du métier et peuvent être facilement trouvées dans la littérature (voir par exemple,"Apports nutritionnels conseillés" Dupin, Abraham et Giachetti deuxième édition 1992, Ed. TEC et DOC Lavoisier)

Une telle composition permet, à elle seule, de répondre aux besoins nutritionnels journaliers d'un patient tout en permettant de réduire la synthèse intracellulaire et l'apport extérieur en polyamines. Elle constitue alors un aliment à part entière.

Bien sûr, il pourra être envisagé d'administrer une telle composition non pas en une seule prise mais en plusieurs prises espacées au cours de la même journée. Chaque ration sera alors définie pondéralement de façon à constituer un sous-multiple d'une ration journalière alimentaire d'un être humain et comprendra:
- éventuellement ledit inhibiteur de la synthèse intracellulaire des polyamines à raison de moins de 50/X g et préférentiellement à raison de 1/X à 10/X g,
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain.

X étant un entier compris entre 2 et 8 et correspondant au nombre de rations devant être ingérées par le patient pour satisfaire ses besoins nutritionnels journaliers.

Le nombre de telles rations pourra être choisi de façon à répondre totalement aux besoins alimentaires journaliers du patient ou bien être choisi de façon à ne couvrir qu'une partie des besoins nutritionnels de celui-ci, le reste de ces besoins étant assurés par un alimentation naturelle pauvre en polyamines (jambon et pâtes ou riz par exemple).

Dans ce cas la composition alimentaire sera utilisée en tant que complément alimentaire.

Les inventeurs ont effectués différents travaux permettant d'établir chez le rat que l'utilisation d'un régime pauvre en polyamines permettait de lutter contre la sensibilisation et la mémorisation de la douleur, et par voie de conséquence la chronicisation de la douleur, mais également de restaurer l'efficacité de la morphine et des autres substances classées comme morphinomimétiques.
- Les figures 1a, 2a et 2b, 4a et 4b représentent des graphes relatifs aux différents résultats du test de Randal et Sellito modifié mis en oeuvre par les inventeurs ;
- la figure 1b, 2c et 2d, 4c et 4d représente des graphes relatifs aux résultats du test « de déséquilibre postural » mis en oeuvre par les inventeurs ;
- la figure 3 représente un gel électrophorétique et un graphe traduisant l'influence de la consommation d'un régime pauvre en polyamines sur le niveau de phosphorylation du résidu tyrosine de la sous-unité NR2B du récepteur NMDA.

Sur la base des mécanismes d'action possibles des polyamines au niveau des récepteurs NMDA, les inventeurs ont établi qu'en activant les sites polyamines de la fraction NR1 ou encore en modifiant la configuration spatiale d'une boucle de 21 acides aminés constitutive de cette sous-unité (cassette N1) pouvant se comporter comme un analogue de polyamine, les polyamines extra cellulaires jouaient un rôle critique dans le développement de la phosphorylation de la sous-unité NR2B des récepteurs NMDA induite par des influx nociceptifs. Ils ont donc recherché si une réduction des sources exogènes de polyamines avait un impact spécifique sur les processus de sensibilisation et de mémorisation de la douleur.

Dans ce but, des rats Sprague-Dawley ont été nourris avec un aliment solide à très faible teneur en polyamines (moins de 10 µg de polyamines par Kg d'aliment) synthétisé comme précédemment décrit (Kergozien et al., Life Sci. 1996, 58, 2209-15), ceci en accord avec les recommandations de Cheauveau et al. (Arch. Sci. Physiol., 1951, 5, 305-322), et répondant aux besoins nutritionnels journaliers des rongeurs. Sur les dessins ce régime alimentaire est indiqué par « régime pauvre en PA ».

Les contrôles ont été nourris avec le même aliment complémenté avec 54 mg/kg de putrescine, 27 mg/kg de spermidine et 7 mg/kg de spermine selon les concentrations en polyamines présentes dans les aliments standards pour rongeurs. Sur les dessins, ce régime alimentaire est indiqué par « régime normal ».

Parallèlement de la néomycine à raison de 2g/L a été administrées aux rats dans leur eau de boisson.

Dans une première étape, les effets d'un régime pauvre en polyamines sur la sensibilité à la douleur ont été étudiés sur des rats normaux grâce au test classique de pression sur la patte avec mesure du cri émis (Test de Randall et Selitto modifié, Kayser et al., 1990, 508, 329-332).

Aucun changement dans le seuil nociceptif n'a été observé suite à un régime sans polyamines de sept jours. Plus précisément, le seuil nociceptif avant régime pauvre en polyamines a été observé à une pression correspondant à une masse de 280g sur la patte et de 281g après dix jours de régime pauvre en polyamines.

Ainsi, il a été démontré qu'un régime pauvre en polyamines n'exerce pas d'effet analgésique en tant que tel.

Parallèlement, il a été observé que ce régime pauvre en polyamines n'avait pas d'incidence sur l'activité locomotrice spontanée nocturne et diurne des rats testés, évalués selon les enregistrements télémétriques classiques.

Parallèlement, un tel régime n'affecte également pas les capacités de mémorisation des rats, capacités évaluées par des tests classiques de reconnaissance dans l'espace.

Aucun changement de température corporelle ni de poids n'a été observé chez les rats testés.

Ainsi, contrairement aux antagonistes des récepteurs NMDA, les régimes pauvres en polyamines donnés pendant plusieurs jours ne s'accompagnent pas d'effets secondaires psychotomimétiques apparents chez le rat.

Dans une seconde étape, les inventeurs ont étudié les conséquences d'un régime pauvre en polyamines dans le procédé de développement de la sensibilisation à la douleur, en comparant les effets de deux injections successives (0.2ml) d'une solution saline à 1 % de carragénine (CAR) dans le coussinet plantaire des animaux. L'évaluation de la sensibilité à la douleur est effectuée grâce au test de Randall Selitto et le niveau de douleur apprécié par un test original (Test de Déséquilibre postural), qui permet d'apprécier quantitativement les changements d'appuis posturaux sur les pattes arrières du rat grâce à un système de jauge de contrainte (Rivat et al., 2002, World Congress on Pain Abstract, 10, 381-382). L'avantage de ce test réside dans le fait qu'il permet, grâce à l'évaluation quantitative d'un comportement antalgique (déséquilibre postural sur les pattes postérieures), d'évaluer un niveau de douleur spontanée ne requérant pas la mise en oeuvre de stimuli nociceptifs expérimentaux brefs et discontinus comme dans le cas de la plupart des test classiques qui ne font qu'évaluer une sensibilité à la douleur (seuil nociceptif). Un second avantage de ce test est qu'il permet d'évaluer quantitativement l'efficacité réelle d'un traitement supposé antalgique tout comme cela peut être fait chez l'homme en clinique.

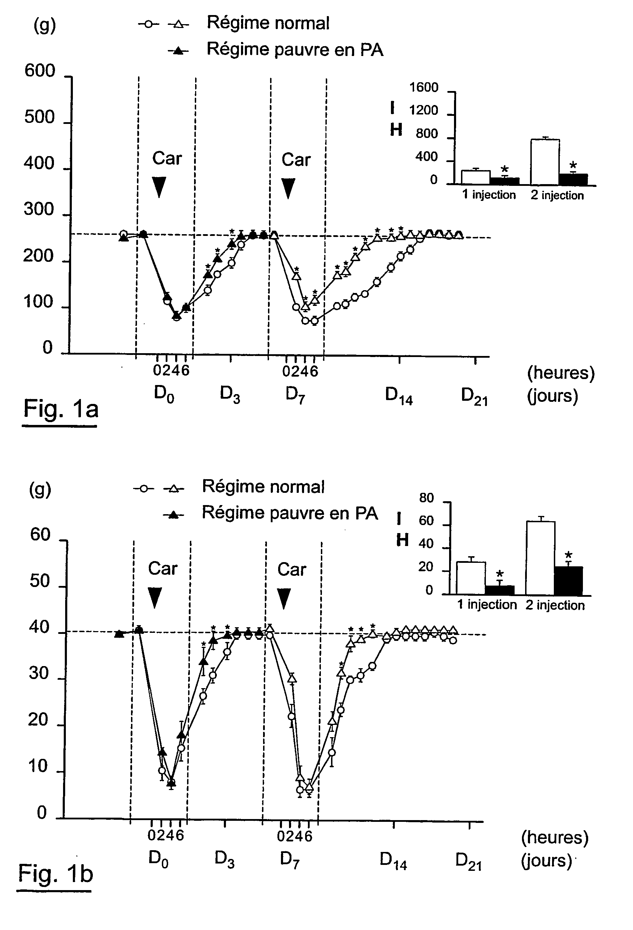

Comme représenté sur la figure 1a et sur la figure 1 b, aucun effet sur le seuil nociceptif ni sur le niveau de douleur n'a été relevé au cours de la première injection de carragénine (« D0 », soit J0 chez les rats ayant eu un régime pauvre en polyamines pendant sept jours avant l'inflammation). Toutefois, lorsque le régime pauvre en polyamines a été donné pendant sept jours de plus, l'hypersensibilité à la douleur tout comme l'augmentation du niveau de douleur observées durant les jours suivants ont été significativement réduites par rapport à celles observées chez des rats alimentés normalement.

Ainsi, chez lez rats dont le régime pauvre en polyamines a été stoppé le jour avant une deuxième injection de carragénine (fig 1a-« D7 » soit 7^{ème} jour), l'augmentation de l'hyperalgésie (sensibilisation à la douleur) succédant à un deuxième stimulus inflammatoire a été totalement supprimée chez les rats ayant préalablement été soumis à un régime pauvre en polyamines. Le régime pauvre en polyamines prévient donc aussi bien l'hyperalgie associée avec la première qu'avec la seconde injection de carragénine.

Ceci prouve qu'un régime pauvre en polyamines qui est complètement exempt d'effet anti-inflammatoire s'oppose au développement du processus de sensibilisation à la douleur qui résulte régulièrement de stimuli nociceptifs inflammatoires successifs et freine donc la mémorisation.

Dans une troisième étape, l'effet d'un régime pauvre en polyamines a été testé ches des rats sur un modèle de douleur incisionnelle de type chirurgical incisés selon le modèle de Brennan et al. (Pain 1996, 64, 493-501) et traités par un opioïde largement utilisé en clinique humaine (fentanyl).

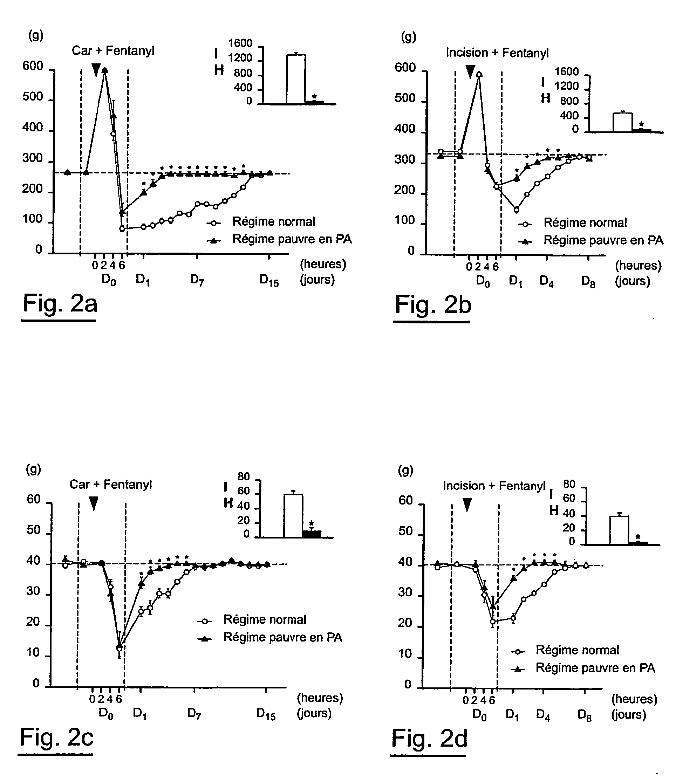

Comme le montre les figures 2a à 2d le régime pauvre en polyamines permet de prévenir totalement le renforcement par le fentanyl de l'hyperalgie de longue durée induite aussi bien par la douleur inflammatoire que par la douleur incisionnelle. Cet effet préventif est observé aussi bien en termes de sensibilité à la douleur (Test de Randall-Selitto, Fig. 2a-b) qu'en termes de niveau de douleur (Test du Déséquilibre postural, Fig2c-d).

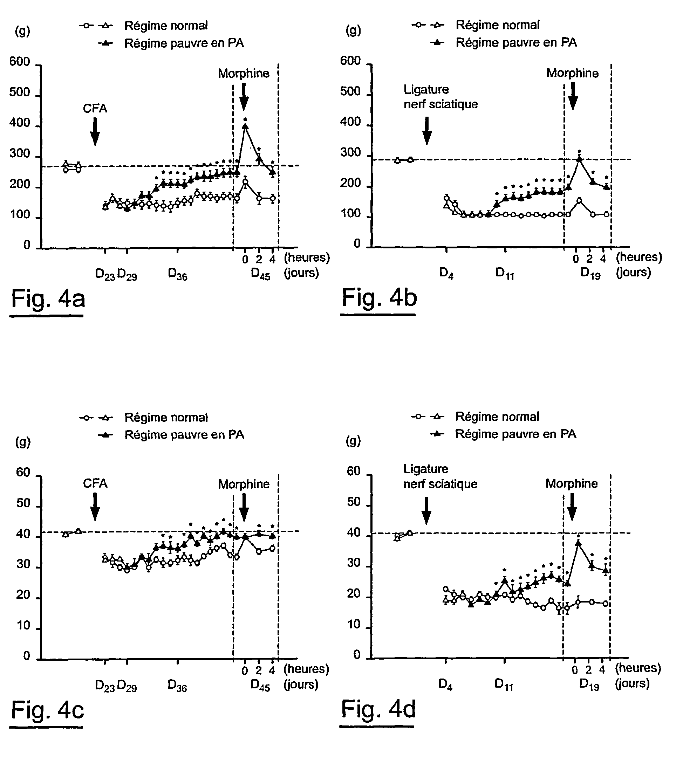

Dans une quatrième étape (voir figure 4a à 4d), des tests ont été effectués sur des rats souffrant de douleurs bien établies de manière durable d'origine monoarthritique provoquées par l'injection d'adjuvent de Freund (CFA)(Butler et al. Pain, 1992, 48, 73-81) ou d'origine neuropathique par ligature du nerf sciatique (Bennet et Xie, Pain, 1988, 33, 87-107). L'efficacité de faibles doses de morphine a été également évaluée dans ces modèles animaux en sachant que les douleurs neuropathiques, bien que non complètement résistantes à la morphine, sont reconnues comme étant très peu sensible à la morphine ou à ses dérivés.

Ces tests montrent que la sensibilité à la douleur (Seuil nociceptif, Test de Randall-Selitto) et la sensation de douleur (Test de Déséquilibre postural) étaient réduites de façon importante en quelques jours chez les rats soumis à un régime pauvre en polyamines. De plus, le régime pauvre en polyamines a amélioré l'efficacité de doses faibles de morphine, particulièrement chez les rats présentant des douleurs neuropathiques.

On peut penser qu'il sera possible, grâce à l'utilisation des compositions pauvres en polyamines de l'invention, de restaurer chez l'homme l'effet analgésique des substances opioïdes telles que la morphine

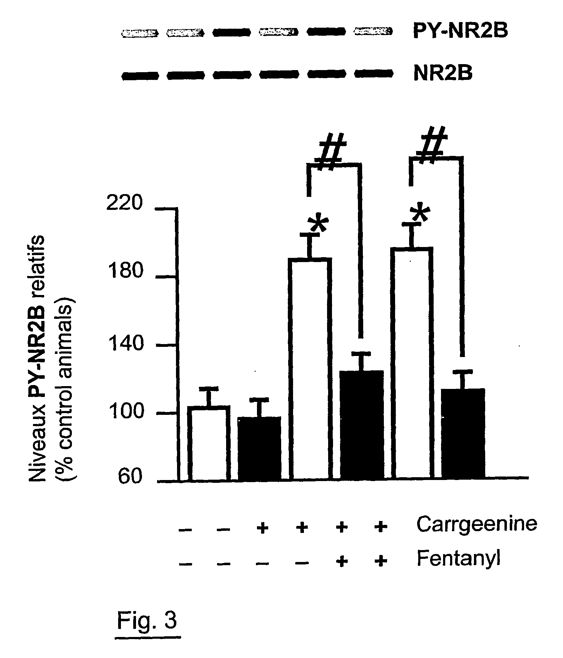

En référence à la figure 3, les protéines de la moelle épinière (région L4-L5) ont été extraites du rachis lombaire chez des animaux soumis ou non à un stimulus inflammatoire, ayant ou non été nourris avec un régime pauvre en polyamines, et ayant ou non été traités par du fentanyl. Les spots situés dans la partie supérieure de l'électrophorèse (1) révèlent les bandes protéiques correspondant aux protéines extraites par immunoprécipitation en présence d'anticorps anti-NR2B, puis révélées en présence d'anticorps anti-phosphotyrosine PY-99. Les spots situés dans la partie inférieure (2) montrent l'immunoréactivité des protéines extraites comme en (1), cette fois révélées par des anticorps anti-NR2B, mais ces dernières ayant préalablement réagi avec des anticorps anti-phosphotyrosine PY-99. Les histogrammes expriment l'intensité de phosphorylation du résidu tyrosine de la sous-unité NR2B du récepteur NMDA (1), par rapport à (2), ceci au cours des différents traitements. Les colonnes en clair correspondent aux animaux témoins, nourris avec un aliment contenant des polyamines (comme décrit précédemment) ; les colonnes en noirs correspondent aux animaux ayant consommé un régime pauvre en polyamines. Les résultats sont exprimés en moy ± SEM. (*) p < 0.05.

Ainsi, le régime pauvre en polyamines a réduit totalement, au niveau spinal, l'augmentation de la phosphorylation de la sous-unité NR2B des récepteurs NMDA induite par la douleur de type inflammatoire provoquée par l'injection de carragénine

En réduisant la composante hypersensibilité à la douleur liée à la phosphorylation de la sous-unité NR2B des récepteurs NMDA, sans éliminer la douleur elle-même, les compositions pauvres en polyamines faisant l'objet de l'invention, peuvent être utilisées dans le cadre de thérapies nutrionnelles non invasives, capables d'améliorer à long terme la prise en charge de différents types de syndromes douloureux.

## Revendications

1. Utilisation d'une composition alimentaire à usage humain présentant moins de 1600 picomoles de polyamines pour la réalisation d'un aliment thérapeutique destiné à lutter contre un syndrome dans lequel la sous-unité NR2-B du récepteur N-méthyl-D-aspartate est impliquée et choisi dans le groupe constitué par :
- la sensibilisation, la mémorisation et par voie de conséquence la chronicisation de la douleur,
- la tolérance aux effets analgésiques des analgésiques opioïdes,
- la dépendance vis-à-vis de substances à potentialité toxicomanogène,
ladite composition contenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

2. Utilisation selon la revendication 1 **caractérisée en ce que** ladite composition contient moins d'environ 400 picomoles/g de putrescine, moins d'environ 400 picomoles/g de spermidine, moins d'environ 400 picomoles/g de spermine et moins d'environ 400 picomoles/g de cadavérine.

3. Utilisation selon l'une des revendications précédentes **caractérisée en ce que** ladite composition contient moins d'environ 400, préférentiellement moins d'environ 200, picomoles/g de polyamines.

4. Utilisation selon la revendication 3 **caractérisée en ce que** ladite composition contient moins d'environ 100, préférentiellement moins d'environ 50, picomoles/g de putrescine, moins d'environ 100, préférentiellement moins d'environ 50, picomoles/g de spermidine, moins d'environ 100, préférentiellement moins d'environ 50, picomoles/g de spermine et moins d'environ 100, préférentiellement moins d'environ 50, picomoles/g de cadavérine.

5. Utilisation selon l'une des revendications précédentes **caractérisée en ce que** ladite composition est enrichie avec au moins un inhibiteur de la synthèse intracellulaire des polyamines à raison d'au plus 15 % en poids par rapport au poids sec total de la composition.

6. Utilisation selon la revendication 5 **caractérisée en ce que** ladite composition est enrichie avec ledit inhibiteur à raison de 0,2 % à 7 % en poids par rapport aù poids sec total de la composition.

7. Utilisation selon la revendication 6 **caractérisée en ce que** ledit inhibiteur de ladite composition est un inhibiteur compétitif de l'ornithine décarboxylase.

8. Utilisation selon la revendication 7 **caractérisée en ce que** ledit inhibiteur compétitif de ladite composition est l'α-méthylornithine.

9. Utilisation selon l'une quelconque des revendications précédentes **caractérisée en ce que** ladite composition contient au moins un antibiotique.

10. Utilisation selon l'une quelconque des revendications précédentes **caractérisée en ce que** lesdits glucides de la composition appartiennent au groupe comprenant les polymères de glucose, les maltodextrines, le saccharose, les amidons modifiés, le glucose monohydraté, le sirop de glucose déshydraté, le monostéarate de glycérol et leurs mélanges.

11. Utilisation selon l'une quelconque des revendications précédentes **caractérisée en ce que** lesdites protéines de ladite composition appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélange.

12. Utilisation selon l'une quelconque des revendications précédentes **caractérisée en ce que** lesdits lipides de ladite composition appartiennent au groupe comprenant l'huile de beurre, l'huile d'arachide, les triglycérides à chaîne moyenne, l'huile de pépins de raisin, l'huile de soja, l'huile d'onagre et leurs mélanges.

13. Utilisation selon l'une quelconque des revendications précédentes **caractérisée en ce que** lesdits lipides de ladite composition sont constitués par un mélange d'au moins une huile d'origine animal, d'au moins une huile d'origine végétale et de stéarate de glycérol.

14. Utilisation selon l'une quelconque des revendications précédentes **caractérisée en ce que** ladite composition constitue la ration journalière alimentaire d'un être humain et **en ce qu'**elle comprend :
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

15. Utilisation selon l'une quelconque des revendications 5 à 14 **caractérisée en ce que** ladite composition constitue la ration journalière alimentaire d'un être humain et **en ce qu'**elle comprend :
- moins de 50 g et, préférentiellement, entre 1 à 10 g dudit inhibiteur de la synthèse intracellulaire des polyamines,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

16. Utilisation selon l'une quelconque des revendications 5 à 13 **caractérisée que** ladite composition est un sous-multiple d'une ration journalière alimentaire d'un être humain et en ce qu'elle comprend :
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain, et
X étant un entier compris entre 2 et 8 et correspondant au nombre de rations devant être ingérées par le patient pour satisfaire ses besoins nutritionnel journaliers.

17. Utilisation selon l'une quelconque des revendications 5 à 14 **caractérisée en ce que** ladite composition est un sous-multiple d'une ration journalière alimentaires d'un être humain et **en ce qu'**elle comprend :
- moins de 50/X g et, préférentiellement, entre 1/X et 10/X g dudit inhibiteur de la synthèse intracellulaire des polyamines,
- entre 75/X g et 500/X g de glucides,
- entre 20/X g et 1,85/X g de lipides,
- entre 20/X g et 225/X g de protéines,
- des vitamines, des minéraux et des électrolytes en quantités suffisantes pour répondre partiellement aux besoins nutritionnels journaliers d'un être humain, et
X étant un entier compris entre 2 et 8 et correspondant au nombre de rations devant être ingérées par le patient pour satisfaire ses besoins nutritionnels journaliers.

18. Utilisation selon l'une quelconque des revendications précédentes **caractérisée en ce que** ladite composition se présente sous une forme sèche à dissoudre extemporanément dans un véhicule neutre.

19. Utilisation selon l'une quelconque des revendications précédentes **caractérisée en ce que** ladite composition inclut un véhicule neutre la rendant prête à l'emploi.

## Claims

1. Use of a food composition for human consumption containing less than 1,600 picomoles of polyamines to make a therapeutic food intended to combat a syndrome in which the NR2-B sub-unit of the N-methyl-D-aspartate receptor is involved and chosen from the group comprising:
- increased sensitivity and memorisation of pain, and consequently the development of chronic pain,
- tolerance to the analgesic effects of opioid analgesics,
- addition to substances with a toxicomanogenic potential,
said composition including, as a percentage of the total dry weight, 10 to 35% of lipids, 8 to 30% of proteins, 35 to 80% of glucides and up to 10% of a mixture composed of vitamins, minerals and electrolytes.

2. Use according to Claim 1, **characterised in that** the said composition contains less than about 400 picomoles/g of putrescine, less than about 400 picomoles/g of spermidine, less than about 400 picomoles/g of spermine and less than about 400 picomoles/g of cadaverine.

3. Use according to any one of the preceding claims, **characterised in that** the said composition contains less than about 400 and preferably less than about 200 picomoles/g of polyamines.

4. Use according to Claim 3, **characterised in that** the said composition contains less than about 100 and preferably less than about 50 picomoles/g of putrescine, less than about 100 and preferably less than about 50 picomoles/g of spermidine, less than about 100 and preferably less than about 50 picomoles/g of spermine, and less than about 100 and preferably less than about 50 picomoles/g of cadaverine.

5. Use according to any one of the preceding claims, **characterised in that** the said composition is enriched with at least one inhibitor of intracellular synthesis of polyamines, with a content by weight not exceeding 15% of the total dry weight of the composition.

6. Use according to Claim 5, **characterised in that** the said composition is enriched with the said inhibitor with a content by weight of between 0.2% and 7% of the total dry weight of the composition.

7. Use according to Claim 6, **characterised in that** the said inhibitor of the said composition is a competitive inhibitor of the ornithine decarboxylase.

8. Use according to Claim 7, **characterised in that** the said competitive inhibitor of the said composition is α-methylornithine.

9. Use according to any one of the preceding claims, **characterised in that** the said composition contains at least one antibiotic.

10. Use according to any one of the preceding claims, **characterised in that** the said glucides of the composition belong to the group comprising glucose polymers, maltodextrines, saccharose, modified starches, monohydrated glucose, dehydrated glucose syrup, glycerol monostearate and mixtures thereof.

11. Use according to any one of the preceding claims, **characterised in that** the said proteins of the said composition belong to the group comprising soluble proteins of milk, soya proteins, serum peptides, powdered egg yolk, potassium caseinate, non-phosphorylated peptides, casein peptides, mixed caseinate, soya isolate and mixtures thereof.

12. Use according to any one of the preceding claims, **characterised in that** the said lipids of the said composition belong to the group including butter oil, peanut oil, medium-chain triglycerides, grape seed oil, soya oil, onagra oil and mixtures thereof.

13. Use according to any one of the preceding claims, **characterised in that** the said lipids of the said composition are composed of a mix of at least one animal oil, at least one vegetable oil and glycerol stearate.

14. Use according to any one of the preceding claims, **characterised in that** the said composition forms a daily food ration for a human being and includes:
- between 75 g and 500 g of glucides,
- between 20 g and 185 g of lipids,
- between 20 g and 225 g of proteins,
- sufficient quantities of vitamins, minerals and electrolytes to satisfy the daily nutritional needs of a human being.

15. Use according to any one of Claims 5 to 14, **characterised in that** the said composition forms a daily food ration for a human being and includes:
- less than 50 g and preferably between 1 and 10 g of the said inhibitor of intracellular synthesis of polyamines,
- between 75 g and 500 g of glucides,
- between 20 g and 185 g of lipids,
- between 20 g and 225 g of proteins,
- sufficient quantities of vitamins, minerals and electrolytes to satisfy the daily nutritional needs of a human being.

16. Use according to any one of Claims 5 to 13, **characterised in that** the said composition is a sub-multiple of a daily food ration for a human being and **in that** it includes:
- between 75/X g and 500/X g of glucides,
- between 20/X g and 185/X g of lipids,
- between 20/X g and 225/X g of proteins,
- sufficient quantities of vitamins, minerals and electrolytes to partially satisfy the daily nutritional needs of a human being, and
X being an integer between 2 and 8 corresponding to the number of rations to be ingested by the patient to satisfy his/her daily nutritional needs.

17. Use according to any one of Claims 5 to 14, **characterised in that** the said composition is a sub-multiple of a daily food ration for a human being and **in that** it includes:
- less than 50/X g and preferably between 1/X and 10/X g of the said inhibitor of intracellular synthesis of polyamines,
- between 75/X g and 500/X g of glucides,
- between 20/X g and 185/X g of lipids,
- between 20/X g and 225/X g of proteins,
- sufficient quantities of vitamins, minerals and electrolytes to partially satisfy the daily nutritional needs of a human being, and
X being an integer between 2 and 8 corresponding to the number of rations to be ingested by the patient to satisfy his/her daily nutritional needs.

18. Use according to any one of the preceding claims, **characterised in that** the said composition is presented in dry form to be extemporaneously dissolved in a neutral vehicle.

19. Use according to any one of the preceding claims, **characterised in that** the said composition includes a neutral vehicle making it ready for use.

## Patentansprüche

1. Verwendung einer Nahrungsmittelzusammensetzung für den menschlichen Verzehr, die weniger als 1.600 Picomol Polyamine aufweist, zur Herstellung eines therapeutischen Nahrungsmittels, das dazu dient, ein Syndrom zu bekämpfen, an dem die Untereinheit NR2-B des N-Methyl-D-Aspartat-Rezeptors beteiligt ist, und das ausgewählt ist aus der Gruppe bestehend aus:
- Schmerzsensibilisierung, Schmerzerinnerung und in der Folge Schmerzchronifizierung,
- Toleranz gegenüber den schmerzlindernden Wirkungen von opioiden Schmerzmitteln,
- Abhängigkeit von Substanzen mit suchterzeugendem Potential,
wobei die Zusammensetzung, in Prozent des Trockengewichts bezogen auf das gesamte Trockengewicht, enthält: 10 % bis 35 % Lipide, 8 % bis 30 % Proteine, 35 % bis 80 % Kohlenhydrate, bis zu 10 % eines Gemischs, bestehend aus Vitaminen, Mineralstoffen und Elektrolyten.

2. Verwendung nach Anspruch 1, **dadurch gekennzeichnet, daß** die Zusammensetzung weniger als etwa 400 Picomol/g Putrescin, weniger als etwa 400 Picomol/g Spermidin, weniger als etwa 400 Picomol/g Spermin und weniger als etwa 400 Picomol/g Cadaverin enthält.

3. Verwendung nach einem der vorhergehenden Ansprüche, **dadurch gekennzeichnet, daß** die Zusammensetzung weniger als etwa 400, vorzugsweise weniger als etwa 200 Picomol/g Polyamine enthält.

4. Verwendung nach Anspruch 3, **dadurch gekennzeichnet, daß** die Zusammensetzung weniger als etwa 100, vorzugsweise weniger als etwa 50 Picomol/g Putrescin, weniger als etwa 100, vorzugsweise weniger als etwa 50 Picomol/g Spermidin, weniger als etwa 100, vorzugsweise weniger als etwa 50 Picomol/g Spermin und weniger als etwa 100, vorzugsweise weniger als etwa 50 Picomol/g Cadaverin enthält.

5. Verwendung nach einem der vorhergehenden Ansprüche, **dadurch gekennzeichnet, daß** die Zusammensetzung mit mindestens einem Inhibitor der intrazellulären Polyaminsynthese mit einem Anteil von höchstens 15 Gew.-%, bezogen auf das Gesamttrockengewicht der Zusammensetzung, angereichert ist.

6. Verwendung nach Anspruch 5, **dadurch gekennzeichnet, daß** die Zusammensetzung mit dem Inhibitor mit einem Anteil von 0,2 Gew.-% bis 7 Gew.-%, bezogen auf das Gesamttrockengewicht der Zusammensetzung, angereichert ist.

7. Verwendung nach Anspruch 6, **dadurch gekennzeichnet, daß** der Inhibitor der Zusammensetzung ein kompetitiver Inhibitor der Ornithin-Decarboxylase ist.

8. Verwendung nach Anspruch 7, **dadurch gekennzeichnet, daß** der kompetitive Inhibitor der Zusammensetzung α-Methylornithin ist.

9. Verwendung nach einem der vorhergehenden Ansprüche, **dadurch gekennzeichnet, daß** die Zusammensetzungen mindestens eins Antibiotikum enthält.

10. Verwendung nach einem der vorhergehenden Ansprüche, **dadurch gekennzeichnet, daß** die Kohlenhydrate der Zusammensetzung zur Gruppe gehören, die Glucosepolymere, Maltodextrine, Saccharose, modifizierte Stärken, Glucosemonohydrat, dehydrierten Glucosesirup, Glycerolmonostearat und deren Gemische umfaßt.

11. Verwendung nach einem der vorhergehenden Ansprüche, **dadurch gekennzeichnet, daß** die Proteine der Zusammensetzung zur Gruppe gehören, die lösliche Milchproteine, Sojaproteine, Serumpeptide, pulverförmiges Eigelb, Kaliumcaseinat, nicht phosphorylierte Peptide, Caseinpeptide, Mischcaseinat, Sojaisolat und deren Gemische umfaßt.

12. Verwendung nach einem der vorhergehenden Ansprüche, **dadurch gekennzeichnet, daß** die Lipide der Zusammensetzung zur Gruppe gehören, die Butteröl, Erdnußöl, mittelkettige Triglyceride, Weintraubenkernöl, Sojaöl, Onagraöl und deren Gemische umfaßt.

13. Verwendung nach einem der vorhergehenden Ansprüche, **dadurch gekennzeichnet, daß** die Lipide der Zusammensetzung aus einem Gemisch mindestens eine Öls tierischen Ursprungs, mindestens eines Öls pflanzlichen Ursprungs und Glycerolstearat bestehen.

14. Verwendung nach einem der vorhergehenden Ansprüche, **dadurch gekennzeichnet, daß** die Zusammensetzung die tägliche Nahrungsmittelration eines Menschen bildet, und **dadurch**, daß sie umfaßt:
- zwischen 75 g und 500 g Kohlenhydrate,
- zwischen 20 g und 185 g Lipide,
- zwischen 20 g und 225 g Proteine,
- Vitamine, Mineralstoffe und Elektrolyte in ausreichenden Mengen, um den täglichen Nährstoffbedarf eines Menschen zu decken.

15. Verwendung nach einem der Ansprüche 5 bis 14, **dadurch gekennzeichnet, daß** die Zusammensetzung die tägliche Nahrungsmittelration eines Menschen bildet, und **dadurch**, daß sie umfaßt:
- mindestens 50 g und vorzugsweise zwischen 1 und 10 g des Inhibitors der intrazellulären Polyaminsynthese,
- zwischen 75 g und 500 g Kohlenhydrate,
- zwischen 20 g und 185 g Lipide,
- zwischen 20 g und 225 g Proteine,
- Vitamine, Mineralstoffe und Elektrolyte in ausreichenden Mengen, um den täglichen Nährstoffbedarf eines Menschen zu decken.

16. Verwendung nach einem der Ansprüche 5 bis 13, **dadurch gekennzeichnet, daß** die Zusammensetzung ein ganzzahliger Teiler der täglichen Nahrungsmittelration eines Menschen ist, und **dadurch**, daß sie umfaßt:
- zwischen 75/X g und 500/X g Kohlenhydrate,
- zwischen 20/X g und 185/X g Lipide,
- zwischen 20/X g und 225/X g Proteine,
- Vitamine, Mineralstoffe und Elektrolyte in ausreichenden Mengen, um den täglichen Nährstoffbedarf eines Menschen teilweise zu decken; und
wobei X eine ganze Zahl im Bereich zwischen 2 und 8 ist und der Anzahl an Rationen entspricht, die von dem Patienten aufgenommen werden müssen, um seinen täglichen Nährstoffbedarf zu befriedigen.

17. Verwendung nach einem der Ansprüche 5 bis 14, **dadurch gekennzeichnet, daß** die Zusammensetzung ein ganzzahliger Teiler der täglichen Nahrungsmittelration eines Menschen ist, und **dadurch**, daß sie umfaßt:
- weniger als 50/X g und vorzugsweise zwischen 1/X und 10/X g des Inhibitors der intrazellulären Polyaminsynthese,
- zwischen 75/X g und 500/X g Kohlenhydrate,
- zwischen 20/X g und 185/X g Lipide,
- zwischen 20/X g und 225/X g Proteine,
- Vitamine, Mineralstoffe und Elektrolyte in ausreichenden Mengen, um den täglichen Nährstoffbedarf eines Menschen teilweise zu decken; und
wobei X eine ganze Zahl im Bereich zwischen 2 und 8 ist und der Anzahl an Rationen entspricht, die von dem Patienten aufgenommen werden müssen, um seinen täglichen Nährstoffbedarf zu befriedigen.

18. Verwendung nach einem der vorhergehenden Ansprüche, **dadurch gekennzeichnet, daß** die Zusammensetzung eine trockene Form aufweist, die unmittelbar vor der Verwendung in einem neutralen Vehikel aufzulösen ist.

19. Verwendung nach einem der vorhergehenden Ansprüche, **dadurch gekennzeichnet, daß** die Zusammensetzung ein neutrales Vehikel beinhaltet, das sie gebrauchsfertig macht.
